# EUROPEAN PATENT SPECIFICATION

(11) **EP 4 240 788 B1**
(45) Date of publication and mention of the grant of the patent: **17.04.2024**
(21) Application number: 21840100.8
(22) Date of filing: 15.12.2021
(51) Int. Cl.: C08H 8/00, C12P 7/08, C12P 7/10, C13K 1/02, C13K 1/04

(54) **OBTAINING SUGAR FROM FERMENTED GRAINS**
GEWINNUNG VON ZUCKER AUS FERMENTIERTEN GETREIDEKÖRNERN
OBTENTION DE SUCRE À PARTIR DE GRAINS FERMENTÉS

(30) Priority: 16.12.2020 GB 202019913
(43) Date of publication of application: 13.09.2023
(73) Proprietor: Nova Pangaea Technologies (UK) Limited, Middlesbrough TS10 4RG (GB)
(72) Inventor: HINDLE, Neil, Middlesbrough TS10 4RG (GB)
(74) Representative: Doherty, William
(86) International application number: PCT/GB2021/053306
(87) International publication number: WO 2022/129903

(56) References cited:
- WO-A2-2011/039635
- US-A1- 2009 165 378

## Description

This invention relates to a process for obtaining sugar from fermented grains, by breaking down hemicellulose and cellulose into constituent sugars.

### Background

As explained in EP 2 483 331B (Nova Pangaea), there are environmental problems that arise from the use of fossil fuels, so that use of biomass as a source for fuel and organic chemicals would be advantageous. Woody or lignocellulosic biomass is largely composed of hemicellulose, cellulose and lignin. Cellulose is principally comprised of C6 sugars while hemicellulose comprises both C5 and C6 sugars. Lignin is a complex polymer which gives physical strength to the biomass but which is tightly bound to the other components. Consequently, it is not straightforward to remove the sugars from the remainder of the biomass. EP 2 483 331B teaches a method of fractionating lignocellulosic biomass by a sequence of steps. Biomass may be fed into a hemicellulose hydrolysis reactor to hydrolyse hemicellulose, so a liquid component includes the products of hemicellulose hydrolysis for example in water, and so that the remaining solid component includes cellulose and lignin. The remaining solid component is then fed to a cellulose hydrolysis reactor which may apply steam at a temperature of between about 400° and 550°C, so as to hydrolyse cellulose and vaporise the resulting sugars; and then condensing the resulting vapours. The remaining solids may be in the form of a lignin char.

EP 2 483 331B envisages the potential use of a wide range of different types of biomass, including wood, corn, straw, grass and other cellulose wastes; and indicates that the material may be pretreated for example by drying, and by comminution to create chips or flakes, indicating a preferred size of flakes being of thickness between 1 mm and 3 mm. In addition, where appropriate, the material may be pretreated to remove volatile components such as natural oils. The hemicellulose hydrolysis reactor may treat the material using steam at a temperature for example between 170° and 250°C and at an elevated pressure for example between 10 bar(a) and 35 bar(a).

These conditions are described in EP 2 483 331B as being sufficient to hydrolyse hemicellulose while minimising degradation of the biomass material. The sugars produced by hemicellulose hydrolysis will dissolve in water, and may be removed from the biomass using additional water and a counter current water flow; use of a screw press to remove liquid is also mentioned. The resulting solid material consists primarily of cellulose and lignin.

EP 2 483 331B goes on to say that the solid material may be subjected to treatment such as drying and a further size reduction, before being subjected to a process to bring about cellulose hydrolysis. This may be achieved using flash thermolysis using superheated steam, which may for example be at a temperature between 350° and 550°C, and for example at a pressure between 1 bar(a) and 2 bar(a), such that the bond between lignin and cellulose is broken and the cellulose is hydrolysed into C6 sugars. The vaporised sugars and any other volatile compounds may then be separated from solid matter, and the vapours condensed to form an aqueous solution.

### The Invention

The production of ethanol from grains of a variety of cereals is a widespread business activity, whether to produce alcoholic beverages such as beer or whisky, or to produce bioethanol for use as a fuel or as an additive to hydrocarbon fuels. This process converts much of the starch in the grains to alcohol, so the resultant fermented grains are depleted in starch. They may be treated as a waste stream, or used as a cattle feed.

For example, "wet cake" is a by-product of the production of bioethanol. This process uses a grain such as sweetcorn or wheat. For example, dry grains of corn are ground, and then mixed with water. The temperature may then be raised and the pH adjusted to a value which suits the enzymes involved in the conversion of starch to sugar; and an amylase enzyme may be added. The mixture may be heated to kill off any undesirable microorganisms, and then cooled to a temperature suitable for enzymes and yeast. The addition of yeast enables fermentation to occur, converting the sugar to ethanol. Once the fermentation has been completed, the mixture is subjected to distillation to separate the ethanol. The remaining mixture is then centrifuged to separate an aqueous liquid phase, which may be referred to as thin stillage, from solids. The resulting wet solids may be referred to as wet cake, which consists of the fermented grain along with some entrained liquid.

The term fermented grain includes brewer's spent grain, which is barley or wheat that has been used to brew beer, and distillers grains which is the corresponding by-product of other alcohol production processes such as whisky or bioethanol, and may comprise wheat, corn or rice grains. As mentioned above, the grain may have been milled or broken up prior to the fermentation. Distillers grains are typically available either wet (WDG), which corresponds to wet cake, or in a dried form after being mixed with concentrated thin stillage, called dried distillers grains with solubles (DDGS).

One effect of the fermentation process is that the proportion of starch in the fermented grain (e.g. in wet cake) is significantly less than in the initial grain. The other constituents of the grain are not significantly affected, so for example whereas the average proportion of protein in corn is about 9%, the proportion of protein in the wet cake is about three times more. The resultant solid matter in the wet cake contains this protein, and also fibre and fat. For example, the composition may be about 30% protein, about 10% fat, and about 50% carbohydrates, including cellulose and hemicellulose, at least partly in the form of fibres, and starch.

Although EP 2 483 331B describes a range of different ways of hydrolysing cellulose, research has shown that there are a number of problems that must be resolved. In particular it has been found that use of superheated steam at a temperature of at least 500°C, for example 550°C, is advantageous in achieving hydrolysis or thermolysis of cellulose, but that there are a number of technical problems in ensuring that this operates consistently, at such a high temperature. For consistent performance it is necessary to ensure a consistent ratio between the mass of steam and the mass of particulate material (which ratio may be called the modulus), so that it is important to ensure that the particulate material is fed into the flow of steam at a consistent rate. For example, if the flow of particulate material is irregular or comes in pulses, while the steam flow is kept constant, this results in uncontrolled reaction conditions, and risks loss of entrainment. Furthermore, it has been found that the particles tend to become sticky when they are an elevated temperature, possibly due to melting of lignin, so it is highly desirable to keep the particles separated from one another as they pass through the reactor, to avoid agglomeration with the formation of large particles, and a build-up of deposits on the walls of the reactor. To a large extent these problems of stickiness can be resolved as long as the individual particles remain entrained in the flow of steam, so for this reason also a consistent flow rate of biomass into the reactor is highly advantageous.

According to the present invention there is provided a process for obtaining sugar from fermented grain, comprising the steps:
(a) treating the fermented grain with hot water to hydrolyse hemicellulose;
(b) separating an aqueous phase containing the products of the hydrolysis of hemicellulose from particulate material;
(c) feeding the particulate material into a flow of steam at above 450°C along a reactor duct to form a flowing mixture of particles and vapours, the mass flow of steam being at least 3 times the mass flow of particulate matter, such that the particulate material is heated rapidly;
(d) then separating at least 75% (by mass) of the particles from the flowing mixture after no more than 3 seconds; and
(e) condensing the vapours from the flowing mixture.

The reaction between the high-temperature steam and the cellulose, which may be referred to as hydrolysis or thermolysis, is typically completed in less than 1 second, and the particles are then no longer sticky, consisting primarily of charred lignin. At this stage the sugars produced by the hydrolysis of cellulose are in vapour form. Downstream of the reactor duct it is therefore necessary to separate the remaining particles from the steam and vapours, and to condense the steam and vapours. Experiments have shown that there is a risk of the formation of blockages, which lead to a build-up of pressure and a backflow of steam; since, as explained above, consistent steam flow is highly desirable, it is important to ensure that the risk of formation of blockages is minimised.

Furthermore, the modulus, that is to say the ratio between the mass flow of steam compared to the mass flow of particulate matter, is at least 3, and may for example be 3.5, 4.0, 4.5, or 5.0. The steam cools as it heats the particulate material, so the resultant temperature of the particles may be in the range 370°C to 450°C. The length of the reactor duct may be such as to ensure that the particles reach the end of the reactor duct in less than 1 s. A steam velocity through the reactor duct of above 8 m/s may be used, more preferably above 10 m/s for example 12 m/s. For example the reactor duct may be of length 6.0 m if the steam velocity is 12 m/s, so that the particles take about 0.5 s to pass through the reactor duct.

As explained above, the fermented grain has a significantly enhanced proportion of protein, as compared to unfermented cereal grain; the proportion in fermented grain may be 30 or 35%, or within this range. It is well known that at elevated temperatures proteins react with sugars in the Maillard reaction, to form polymers. Surprisingly it has been found that in the process of the present invention this reaction does not occur to any significant extent; this may be because the hydrolysis is performed rapidly.

It will be appreciated that the chemical processes that take place when performing the operation referred to here as thermolysis may alternatively be referred to as hydrolysis, pyrolysis, depolymerisation or degradation; the overall result is that cellulose is separated from lignin and is broken down into smaller compounds. In this document the process of treating the material with high temperature steam to create smaller compounds from the cellulose is referred to as cellulose thermolysis, whereas this process was referred to as hydrolysis in EP 2 483 331.

The reactor duct has a longitudinal axis, and may be provided with an eductor at one end of the reactor duct, the eductor comprising an entry chamber and a venturi-shaped exit channel, a nozzle, a sloping deflector plate above the nozzle, and an inlet port through which the particulate material may be fed onto the deflector plate and into the entry chamber, the nozzle and the venturi- shaped exit channel both being aligned with the longitudinal axis of the reactor duct. The superheated steam at a temperature above 450°C is supplied to flow through the nozzle. During operation the superheated steam may be at a temperature of for example 550°C, and may be fed to the nozzle at a pressure of for example less than 1.5 bar(a). The nozzle is therefore at the same temperature. The deflector plate ensures that the particles of the particulate material do not come into contact with the nozzle, but rather are entrained by the flow of steam from the nozzle. The provision of such an eductor ensures the particulate material is fed at a substantially steady rate into the flow of steam.

For example a source of high pressure steam may be available at a pressure of 10 bar(a). In such a case the steam supply to the reactor duct may comprise a pressure-reducing valve to throttle the pressure down to about 1.5 bar(a). The flow rate of the steam through the nozzle is primarily determined by the steam pressure and the restriction imposed by the nozzle, but in addition there may also be a flowmeter, operating in conjunction with an up-stream flow-control valve, to monitor and tightly control the flow rate of the steam. The plant may also include a superheater to ensure the steam reaches the eductor nozzle at the desired temperature, which may be 550°C, and may be at a pressure of 1.3 bar(a); the superheater should be close to the thermolysis reactor, with minimal bends in the steam flow path, and with thermal insulation to minimise heat loss. The superheater may for example be electrically heated.

In one embodiment the particulate material is fed into the inlet port from a hopper using a twin screw conveyor. The twin screw conveyor provides a pressure barrier between the hopper and the reactor, so as to inhibit any backflow of steam, and to prevent material being uncontrollably sucked into the reactor. It is advantageous if the particulate material is of substantially uniform particle size.

A cyclone may be provided downstream of the reactor duct to remove and collect the entrained particles. In this case a feed duct must convey the superheated steam and entrained particles from the downstream end of the reactor duct to the cyclone, feeding the flowing material through an entry port into the cyclone to ensure cyclonic flow. This entry port may for example be of a rectangular shape. Preferably there are no abrupt reductions in the cross-sectional area through which the superheated steam and entrained particles flow between the reactor duct and the input port for the cyclone, and no abrupt changes in flow direction.

The steam and the sugars and other vapours produced by the thermolysis are then condensed. This may use a heat exchanger in which a coolant flows in heat exchanger channels that are in thermal contact through heat-conducting walls with flow channels for the steam and vapours. Alternatively, it may use direct contact with a cooling liquid. By way of example there may be two successive cooling devices, the first acting as a desuperheater, for example by spraying a liquid so as to decrease the vapour temperature to just above the saturation point. The second may be arranged to achieve full or at least partial condensation of all the steam and vapours present, and may for example feed a coolant liquid onto trays so that coolant overflowing from the trays cascades down in counter current to the hot vapours.

Flow of any gases or vapours that are not condensed by the condenser may be ensured by a fan downstream of the condenser, and this will also generate any required under-pressure in the reactor duct. For example there may be some air entrained with the particulate material that is fed into the reactor duct.

The hemicellulose hydrolysis step may treat the fermented grain using steam at a temperature for example between 170° and 250°C and at an elevated pressure for example between 10 bar(a) and 35 bar(a). After washing and drying, the resulting solid material would consist primarily of cellulose and lignin. An acid such as phosphoric or sulphuric acid may be added to the fermented grain before it is subjected to the hemicellulose hydrolysis. It has been found that the hemicellulose hydrolysis step is more effective if an acid is present, and can be carried out at a somewhat lower temperature, for example between 150°C and 180°C. To some extent the hemicellulose hydrolysis reaction may be autocatalytic, because of formation of organic acids such as acetic acid. Nevertheless the addition of an acid prior to starting the hydrolysis is beneficial in ensuring satisfactory hydrolysis at a lower temperature than would otherwise be required.

The separating of the particulate material from the water and water-soluble compounds may involve a filter or press, and may involving a rinsing or washing step. The particulate material may then be dried, to evaporate excess moisture, before being subjected to the superheated steam step to perform cellulose thermolysis; the dried particulate material may also be subjected to a size reduction, for example with a mill, as particles of a more consistent size will be fed more consistently into the reactor duct, and small particles will heat through more quickly in the superheated steam.

The aqueous mixture formed as a result of the washing step contains the soluble products of hemicellulose hydrolysis, which are predominantly C5 sugars (such as xylose), with some C6 sugars (such as glucose and mannose). The products of cellulose thermolysis may be referred to as C6 sugars, but in practice consist of a mixture which may for example contain glucose, levoglucosan, levoglucosenone, oligomeric anhydrosugars, sugar oligomers and sugars chemically bound to phenol derivatives. The proportions may depend on the exact chemical composition of the fermented grain that is being treated, and the type of acid present. The cellulose thermolysis principally produces the volatile compound anhydroglucose, which may be referred to as levoglucosan.

The invention will now be further and more particularly described, by way of example only, and with reference to the accompanying drawings in which:
Figure 1 shows a flow diagram for a process of the invention for treating fermented grain, including a thermolysis reactor, a cyclone and condensers;
Figure 2 shows a schematic longitudinal cross-sectional view of the thermolysis reactor of Figure 1, showing the inlet end of the thermolysis reactor;
Figure 3 shows a flow diagram of the steam supply system for the thermolysis reactor of Figure 1;
Figure 4 shows a perspective view of a hopper to feed particulate matter into the thermolysis reactor of Figure 1;
Figure 5 shows a longitudinal cross-sectional view of the downstream end of the thermolysis reactor;
Figure 6 shows a side view of a screw of the downstream end of the thermolysis reactor;
Figure 7 shows a longitudinal cross-sectional view of the condensers of Figure 1, along with the associated flow diagram; and
Figure 8 shows a perspective view of a tray forming part of one of the condensers of Figure 7.

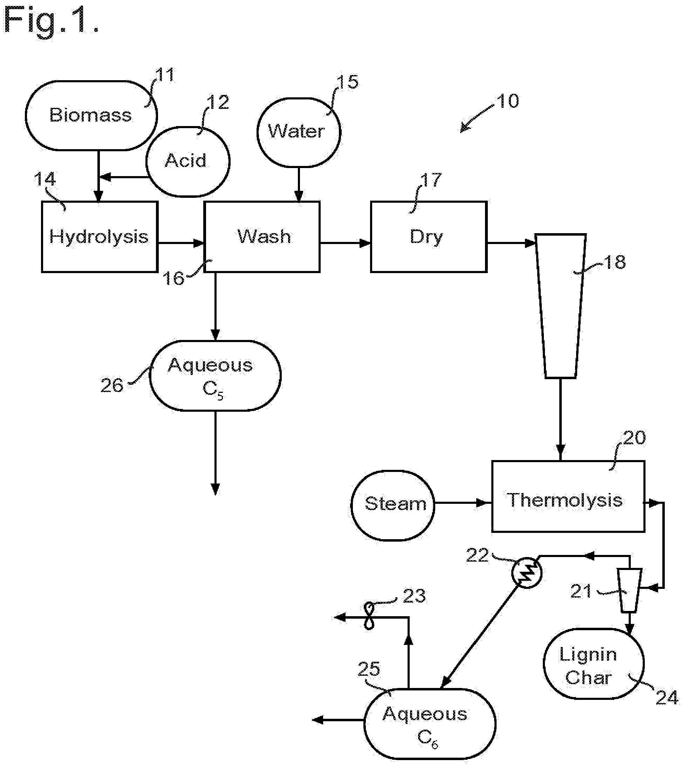

Referring to Figure 1, the present invention provides a process 10 for treating biomass 11 in the form of fermented grain, such as wet cake from a bioethanol process, so as to obtain C5 and C6 sugars by breaking down the hemicellulose and cellulose polymers within the biomass. The process uses two different reaction steps that are performed at different temperatures, and may also be carried out at different pressures, but both use water as the reactant. However, prior to performing the reaction steps the biomass 11 may be chopped or milled into smaller pieces, and may be heated to evaporate any vapours.

After performing any such pre-treatment, the fermented grain biomass 11 is impregnated 12 with a strong acid, for example with dilute sulphuric acid (i.e. about 1 mole/L) typically at a rate of between 1 - 2 wt % of the dry biomass, before being introduced by a screw conveyor into a reactor 14 in which the biomass 11 is contacted with steam/water at a temperature of between 150° and 180°C and a pressure of between 6 bar and 10 bar, for example at 165°C and a pressure of 6.5 bar (gauge); there is little air present. This may be a tube reactor along which the biomass 11 travels along with the high-pressure steam/water. Under these reaction conditions the hemicellulose breaks down mainly to form C5 sugars, with also some C6 sugars, and organic acids, depending on the inherent composition of the biomass 11.

At the operating pressure of 6.5 bar, the water at 165°C is liquid as long as the pressure is maintained. As the material leaves the reactor 14 it may be cooled by depressurisation, exploding the biomass. A proportion of the water evaporates, typically about 10%, cooling the remaining biomass to below 100°C. For example the mixture of biomass and hot water may be released in slugs from the reactor. It is then necessary to remove the liquid phase from the treated biomass before it can be subjected to the second reaction step, both to remove sugars and to remove alkali material and any inorganic acid. Although some of the liquid may be removed by compression, this doesn't enable all the liquid to be expelled. It is therefore preferable to remove the liquid phase by a washing step.

The solid material that has been subjected to the hydrolysis step in the reactor 14 is therefore then washed at step 16, for example using an aqueous solution, and/or clean water, as indicated at step 15.

The washed material is then dried at step 17 to evaporate excess moisture, and may be further comminuted (not shown) to ensure all the particles are small enough to heat up quickly. The material is then introduced into a hopper 18 with a twin screw outlet, and thence into a thermolysis reactor 20 in which the solid material is entrained into a flow of superheated steam at a significantly higher temperature, for example 550°C. The particles of solid material cool the superheated steam while being heated up, and typically reach a final temperature of 370°C to 450°C. The particles are effectively subjected to a temperature in the range for example 370°C to 410°C or 420°C for a short period which may for example be between 0.4 seconds and 1 second. Under these reaction conditions the cellulose undergoes thermolysis, mainly producing C6 sugar derivatives which are volatile under these conditions. After passing through the thermolysis reactor 20 the particulate material, which at this stage is a solid lignin char 24, may be separated from the vapours and gases by passing through a cyclone 21, and the vapours then condensed in a condenser 22. A fan 23 ensures the through-flow of any uncondensed vapours or steam and any non-condensable gases, such as air entrained in the particulate material.

The condensed vapours create an output stream 25 which is an aqueous solution of the products of degradation or thermolysis of the cellulose, which will primarily consist of C6 sugars, typically in an anhydrous form. For example, the products may be anhydroglucose (which is also called levoglucosan), but there may also be phenolic-substituted glucose, and phenolic-substituted anhydrosugars as well as oligomeric anhydrosugars and sugar oligomers.

This output stream 25 of condensed vapours from the cellulose thermolysis reactor 20 may optionally be fed back (not shown) and used in the washing step 16. In that case the resulting aqueous solution 26 contains the C5 sugars produced during hemicellulose hydrolysis, and also the C6 sugars produced during cellulose thermolysis, and at least the latter may be in an anhydrous form. The aqueous solution 26 also contains the bulk of the acid added at step 12 before the biomass was treated in the hemicellulose hydrolysis reactor 14. Alternatively, as indicated in figure 1, the aqueous solution of output stream 25 containing the products from cellulose thermolysis and the aqueous solution 26 containing the products of hemicellulose thermolysis may be treated as separate product streams.

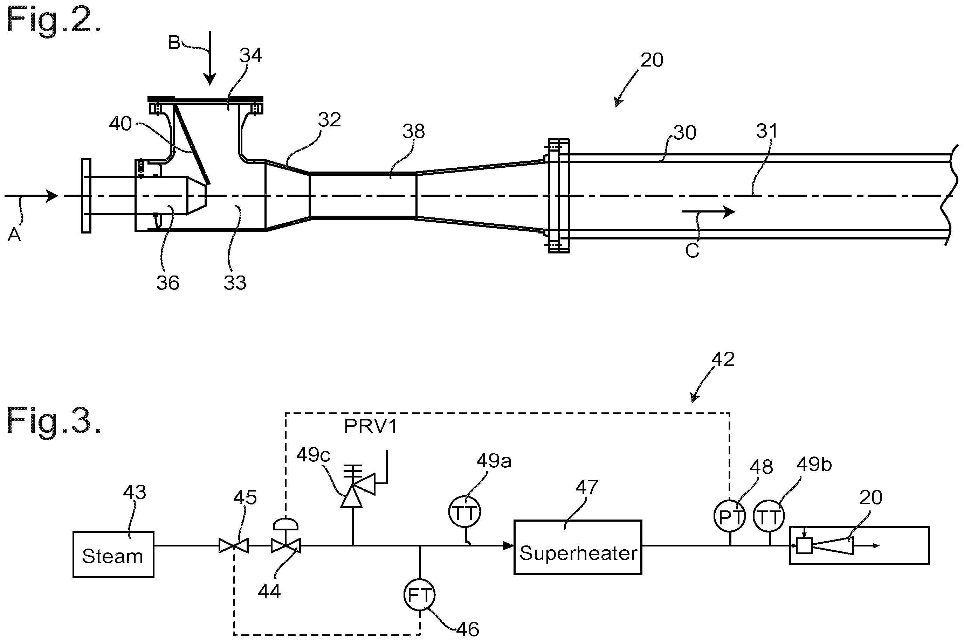

Referring now to Figure 2, which shows a longitudinal view of the thermolysis reactor 20, showing the inlet end of the reactor 20. The reactor 20 comprises a tubular reactor duct 30 with a longitudinal axis 31, which may for example comprise a stainless steel tube of diameter 0.26 m and of length 6.0 m. An eductor 32 is attached to one end of the duct 30. The eductor 32 comprises an entry chamber 33 with an inlet port 34, a nozzle 36 for inflow of steam, and a venturi-shaped exit duct 38 whose diameter narrows down and then broadens out; the end of the venturi-shaped exit duct 38 is attached to one end of the reactor duct 30. The nozzle 36 and the exit duct 38 are aligned with the longitudinal axis 31 of the reactor duct 30. A sloping deflector plate 40 is mounted above the open end of the nozzle 36.

In operation of the thermolysis reactor 20, particulate material from the hopper 18 is fed at a steady rate of 175 kg/h into the inlet port 34, and steam at 550°C is supplied at 700 kg/h through the nozzle 36, corresponding to a modulus of 4. This ensures a steam flow at 12 m/s along the reactor duct 30. The steam is supplied to the nozzle at 130 kPa (1.3 bar(a)), generating a pressure reduction of about 2 kPa (20 mbar) below the inlet port 34. The deflector plate 40 ensures the particles do not contact the hot surfaces of the nozzle 36, but fall down under the influence of the pressure reduction, into the jet of steam from the nozzle 36, and are entrained into the flowing steam. The steam pressure within the entry chamber may be 100 kPa (1.0 bar(a)), and at the other end of the reactor duct 30 the steam pressure drops to about 95 kPa (0.95 bar(a)). Arrow A indicates the steam flow, arrow B represents the flow of particulate material, and arrow C represents the resultant flow of these reagents and of thermolysis products.

Referring now to Figure 3, the steam supply system 42 receives a supply of saturated steam, indicated at 43, at 1.0 MPa (10 bar(a)) from a conventional boiler (not shown). The saturated steam is passed through a pressure reducing valve 44 to throttle the steam down to about 150 kPa (1.5 bar(a)). The flow rate of the steam is primarily defined by the restriction imposed by the nozzle 36, but in addition, in this example, upstream of the pressure reducing valve 44 is a flow control valve 45 which operates in response to a flowmeter 46 to further ensure the required mass flow of steam.

The steam is then passed through a superheater 47 to raise its temperature to 550°C, or slightly above (for example 570°C) if it is necessary to compensate for any significant temperature drop between the outlet of the superheater 47 and the outlet of the nozzle 36. A pressure sensor 48 downstream of the superheater 47 works in conjunction with the pressure reducing valve 44 to ensure that the steam pressure supplied to the nozzle 36 of the eductor 32 is consistently at 130 kPa (1.3 bar(a)).

To monitor and control operation, the steam supply system 42 also includes temperature sensors 49a and 49b to measure the temperature of the steam before and after passage through the superheater 47; and there is also a pressure relief valve 49c which would release pressure if it exceeded a safe value; this may for example be set at 200 kPa (2 bar(a)).

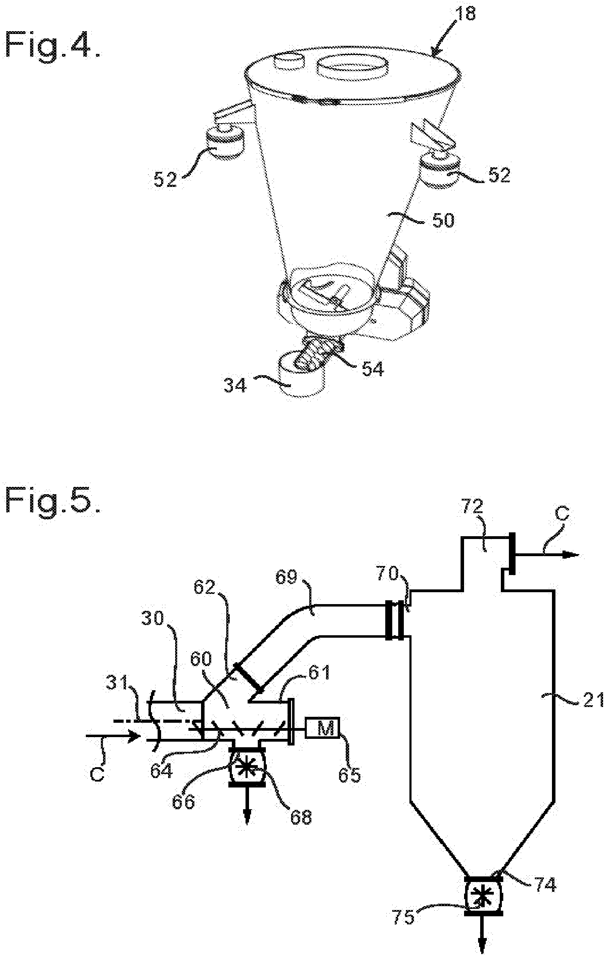

Referring now to Figure 4, the hopper 18 comprises a chamber 50 of tapered form, supported on weight sensors 52 (only two are shown) so the mass of dispensed material can be monitored and controlled. At the base, which is the narrower end of the chamber 50, is a twin screw outlet 54, arranged to feed the particulate material into the inlet port 34 of the eductor 32. The twin screw outlet 54 is enclosed, so minimal air is entrained in the particulate material fed into the inlet port 34 (this enclosure is not shown).

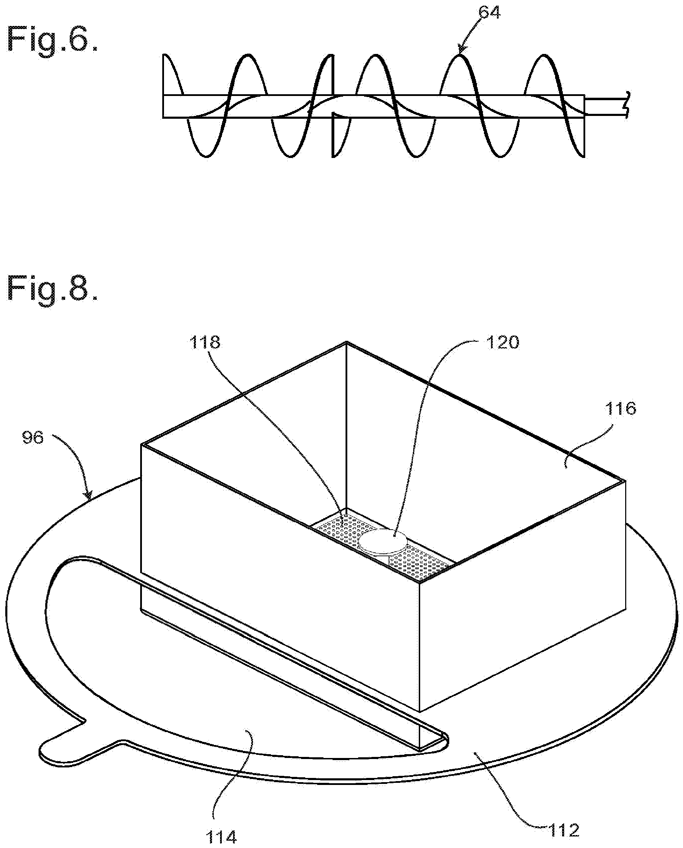

Referring now to Figure 5, this shows the downstream end of the thermolysis reactor 20. The downstream end of the tubular reactor duct 30 is connected to an end fitting 60 which is generally Y -shaped, with a first portion 61 aligned with the longitudinal axis 31 of the tubular reactor duct 30, and with a diverging portion 62 inclined at 135° to the longitudinal axis 31. The cross-sectional area of the diverging portion 62 is substantially the same as that of the tubular reactor duct 30, and provides an unobstructed flow path. The end of the first portion 61 is closed, and within the first portion 61 is mounted a screw 64 driven by an external motor 65. There is an outlet port 66 at the midpoint of the first portion 61, in the bottom wall, and this outlet port 66 is closed by a rotary valve 68. Referring also to Figure 6, the screw 64 has screw flights with a diameter 60% of the tubular reactor duct 30, the flights along the section of the first portion 61 closer to the tubular reactor duct 30 being of opposite hand to those closer to the closed end of the first portion 61, such that rotation of the screw 64 by the motor 65 displaces any particulate matter that settles on the lower walls of the first portion 61 towards the outlet port 66.

Referring again to Figure 5, the diverging portion 62 is joined to a connecting duct 69 which curves back to horizontal and gradually tapers in width (not shown in the side view of the figure), and the cross-section gradually changes to rectangular, being connected at its other end to a rectangular inlet port 70 of the cyclone 21, being aligned so as to feed the gases, vapours and solid particles tangentially into the cyclone 21. Gases and vapours emerge through an outlet port 72 at the top of the cyclone 21, while particulate material collects at the tapered bottom portion of the cyclone 21, where there is an outlet 74 with a rotary valve 75. Substantially all the particulate material, say at least 90% by mass, is either removed by the screw 64 through the outlet port 66 or is trapped by the cyclone 21.

The desired reaction of the thermolysis of cellulose to produce sugars takes place under these conditions very quickly, typically in less than 1 second. If the particulate material is exposed to the reaction conditions (i.e., the high temperature steam) for significantly longer, the lignin will degrade to produce unwanted organic compounds such as phenols, and may also produce tar. It is therefore advantageous to remove the particulate material from contact with the high temperature steam once the desired reaction has taken place. Hence during operation the screw 64 is rotated continuously to transfer any deposited particulate material to the outlet port 66, and the rotary valve 68 is operated continuously to remove any such particulate material and so to prevent further degradation; and similarly the rotary valve 75 at the base of the cyclone 21 is operated continuously to remove the particulate material from the cyclone 21. The rotary valves 68 and 75 significantly inhibit any flow of air into the thermolysis reactor 20 or the cyclone 21.

Operation of the thermolysis reactor 20 leads to the formation of particles of lignin char. Some of these particles may settle out at the end fitting 60, in which case they can be removed through the gate valve 66. Most of the particles are carried along with the high temperature gas and vapour stream along the diverging portion 62, and so are fed through the connecting duct 69 into the cyclone 21. Within the cyclone 21 all but the smallest of particles settle out, so the gas and vapour stream emerging from the cyclone 21 contains only very small particles, if any.

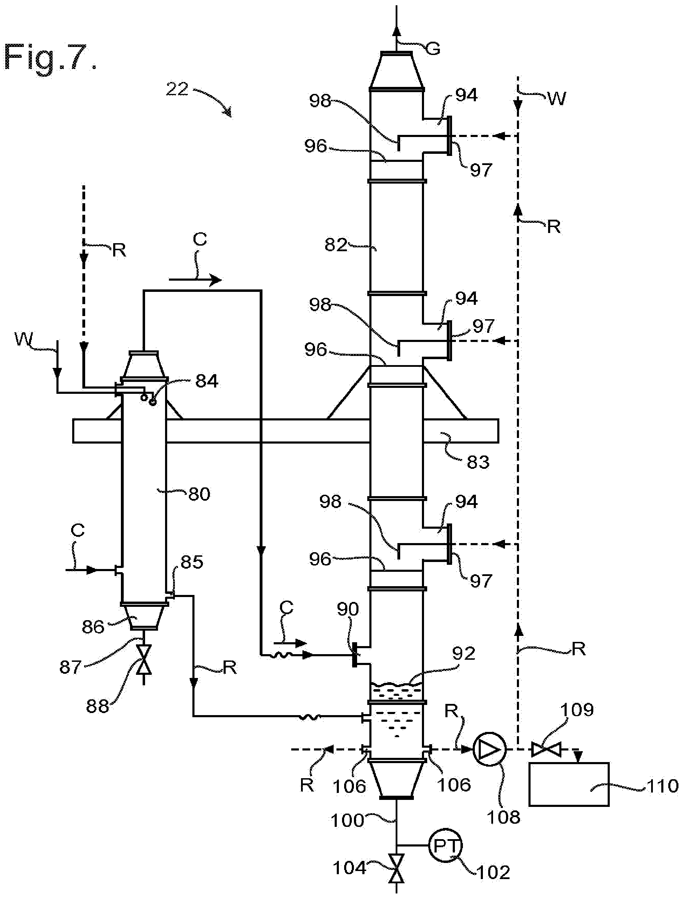

Referring now to Figure 7, the condenser 22 in this case is a two-stage condenser using direct contact with cooling liquid, comprising a first condenser 80 and a second condenser 82 which are both mounted on a support frame 83. The first condenser 80 may act as a de-superheating condenser; it is a vertical cylindrical chamber, the flow of gases, vapours and any remaining particles (as indicated by arrows C) being fed to near the bottom of the chamber to emerge from the top. Water is sprayed into the gas stream from spray nozzles 84 near the top of the chamber; and an outlet 85 near the bottom allows water to be recycled as indicated by arrow R. The bottom portion 86 of the chamber tapers down to an outlet 87 which is usually closed by a valve 88.

The spray nozzles 84 may be fed either with mains water, indicated by arrow W or by recycled water indicated by arrow R. When operation starts, mains water is used, but during steady- state operation the water is primarily recycled water. The quantity of water sprayed is only enough to cool the steam so that it is saturated. This de-superheating condenser 80 will also tend to trap any particles, which will therefore collect in the bottom portion 86, and can occasionally be withdrawn through the valve 88.

The second condenser 82 is a significantly larger vertical cylindrical chamber, and the de-superheated gases and vapours from the first condenser 80, indicated by arrow C, are fed into an inlet port 90 towards the bottom of the chamber, but above the water level 92 during operation. Remaining gases and vapours leave the second condenser 82 from the top, indicated by arrow G, and this flow is ensured by the fan 23 (shown in Figure 1).

The second condenser 82 is provided with three inlet ports 94 spaced apart along its length, each port 94 being a short distance above a horizontal tray 96 within the chamber, each tray 96 being clamped between flanges of tubular elements that form the cylindrical chamber. Each port 94 is closed by an end plate 97 and is provided with a water inlet 98 to feed water onto the tray 96. In operation water is continuously cascading down from the trays 96, so the gas flow is counter current to the falling water. Initially, as indicated by arrow W, the water may be mains water, but during normal operation the water provided to each water inlet 98 is recycled water, indicated by arrow R, which is an aqueous solution of the products produced by the cellulose thermolysis.

During operation the quantity of water provided to the second condenser 82 is such as to ensure that the outgoing gases, indicated by arrow D, remain at a temperature of at least 60°C.

Nevertheless, the bulk of the steam provided to the thermolysis reactor 20 will be condensed during its passage through the second condenser 82.

An outlet duct 100 connects to the bottom of the chamber, and connects to a pressure sensor 102 and to an outlet valve 104 which is normally closed. The recycled water which is provided to both the spray nozzles 84 and the water inlets 98 is taken from outlet ports 106 near the bottom of the chamber, being recirculated for example by a pump 108. During operation the concentration of the products of thermolysis of cellulose in the aqueous solution will at least initially increase as this solution is recirculated, and the total quantity of water will gradually increase. When the liquid level 92 reaches a preset threshold, as sensed by the pressure sensor 102, a valve 109 is opened so that some of the aqueous solution is transferred to a product storage tank 110.

Referring now to Figure 8, one of the trays 96 is shown in perspective view. It is of stainless-steel and consists of a circular plate 112 with a D-shaped aperture 114 on one side, and a rectangular open-topped box 116 alongside it. The rectangular box 116 projects above the plate 112 and slightly below the plate 112, and the box 116 has a removable perforated base plate 118 with multiple perforations; the perforated base plate 118 rests on an internal flange or lip around the bottom of the wall of the box 116. A short rod is welded to the centre of the perforated base plate 118, with a small flat circular disc 120 at its top. Referring also to figure 7, each water inlet 98 feeds water downward onto the circular disc 120 of the corresponding tray 96, so the water spreads out over the perforated base plate 118 to fall through the perforations. The second condenser 82 includes three trays 96, and these are arranged with the D-shaped apertures 114 on alternate sides. During operation, the gases and vapours flow upwards through these D-shaped apertures 114, and because successive trays 96 have their D-shaped apertures 114 on opposite sides the gases and vapours must follow a sinuous path, contacting the falling water.

It has been found that operating at a temperature no lower than 60°C ensures that any tar that may be present, as a potential product of thermolysis, remains sufficiently fluid that it does not form blockages. It will therefore be carried through into the product storage tank 110 along with the aqueous solution, and can subsequently be separated. Nevertheless, if any blockage of the perforations of a tray 96 occurs, the perforated base plate 118 can be replaced during maintenance by disconnecting the end plate 97 from the port 94, lifting out the base plate 118 through the port 94, and replacing it with a clean base plate 118. It will also be appreciated that both the first condenser 80 and the second condenser 82 have a dead volume below the inlet for gases and vapours, to remove any tar or particulate char material, which ensures that such material does not cause blockages to pipework or pumps downstream.

It will be appreciated that the description above is given by way of example only, and that modifications to the process may be made while remaining within the scope of the invention, which is as defined in the claims. By way of example, the flow diagram of Figure 7 indicates that the condensate formed by the first condenser 80 is fed from the outlet 85 to combine with the condensate in the bottom of the second condenser 82. Under some circumstances substantially all the sugar vapour is condensed within the first condenser 80, whereas the bulk of the steam is condensed in the second condenser 82, and under these conditions it may be preferable to keep the condensate from the first condenser 80 separate from the condensate from the second condenser 82, so it may be appropriate to feed the liquid from the outlet 85 to a storage tank corresponding to the product storage tank 110.

As described above, the downstream end of the thermolysis reactor 20 is provided with the generally Y-shaped end fitting 60 in order to accommodate the screw 64, as shown in Figure 5; however, if there is substantially complete entrainment of the particulate matter within the flow of steam, and so negligible deposition onto the walls, then both the screw 64 and the Y-shaped end fitting 60 can be omitted, being replaced by a straight duct leading from the end of the reactor tube 30 to the inlet port 70 of the cyclone 21.

## Claims

1. A process (10) for obtaining sugar from fermented grain, comprising the steps:
(a) treating the fermented grain with hot water to hydrolyse hemicellulose;
(b) separating an aqueous phase containing the products of the hydrolysis of hemicellulose from particulate material;
(c) feeding the particulate material into a flow of steam at above 450°C along a reactor duct (30) to form a flowing mixture of particles and vapours, the mass flow of steam being at least 3 times the mass flow of particulate matter, such that the particulate material is heated rapidly;
(d) then separating at least 75% (by mass) of the particles from the flowing mixture after no more than 3 seconds; and
(e) condensing the vapours from the flowing mixture.

2. A process (10) as claimed in claim 1, wherein the length of the reactor duct (30) and the flow rate of steam, are such as to ensure that the particles reach the end of the reactor duct (30) in less than 1 s.

3. A process (10) as claimed in claim 1 or claim 2, wherein the particulate material is fed into the flow of steam through an eductor (32) at one end of the reactor duct (30), the eductor (32) comprising an entry chamber (33) and a venturi-shaped exit channel (38), a nozzle (36), a sloping deflector plate (40) above the nozzle (36), and an inlet port (34) through which the particulate material may be fed onto the deflector plate (40) and into the entry chamber (33), the nozzle (36) and the venturi-shaped exit channel (38) both being aligned with the longitudinal axis (31) of the reactor duct (30); and wherein the steam at a temperature above 450°C is supplied to flow through the nozzle (36).

4. A process (10) as claimed in claim 3, wherein the steam is supplied at a temperature of above 500°C, and is fed to the nozzle (36) at a pressure of less than 1.5 bar(a).

5. A process (10) as claimed in any one of the preceding claims, wherein the separation of the particles from the flowing mixture is carried out using a cyclone (21) downstream of the reactor duct (30) to remove and collect the entrained particles.

6. A process (10) as claimed in any one of the preceding claims, wherein condensing of the sugars, steam and other vapours is achieved by direct contact with a cooling liquid.

7. A process (10) as claimed in any one of the preceding claims, wherein condensing of the sugars, steam and other vapours is achieved by passage through two successive cooling devices, the first acting as a desuperheater to decrease the vapour temperature to just above the saturation point.

8. A process (10) as claimed in claim 7, wherein the second cooling device is arranged to achieve full or at least partial condensation of all the steam and vapours present.

9. A process (10) as claimed in any one of the preceding claims, wherein the condensing of the sugars, steam and other vapours is achieved by cooling to no lower than 60°C to ensure that any tar that may be present remains sufficiently fluid that it does not form blockages.

10. A process (10) as claimed in any one of the preceding claims, wherein a fan (23) downstream of the condenser (22) is used to achieve gas flow and to ensure a desired pressure in the reactor duct (30).

11. A process (10) as claimed in any one of the preceding claims, wherein the hemicellulose hydrolysis step uses steam/water at a temperature of between 150° and 180°C and a pressure of between 6 bar and 10 bar, for example at 165°C and a pressure of 6.5 bar (gauge), after addition of an acid.

12. A process (10) as claimed in any one of the preceding claims, wherein the separating of the particulate material from the water and water-soluble compounds, after hemicellulose hydrolysis, utilises a filter or press, and may involve a rinsing or washing step (16).

## Patentansprüche

1. Verfahren (10) zur Erzeugung von Zucker aus fermentiertem Getreide mit den folgenden Schritten:
(a) Aufbereitung des fermentierten Getreides mit heißem Wasser zur Hydrolyse der Hemicellulose;
(b) Abscheiden einer wässrigen Phase, die die Produkte der Hydrolyse der Hemicellulose enthält, von partikelförmigem Material;
(c) Zuführen des partikelförmigen Materials in einen Dampfstrom von über 450°C entlang einer Reaktionsleitung (30), um ein strömendes Gemisch aus Partikeln und Dämpfen auszubilden, wobei der Massenstrom an Dampf mindestens das Dreifache des Massenstroms an partikelförmigem Material beträgt, so dass das partikelförmige Material schnell erhitzt wird;
(d) anschließendes Abscheiden von mindestens 75 % (Massenprozent) der Partikel aus dem strömenden Gemisch nach nicht mehr als 3 Sekunden; und
(e) Kondensieren der Dämpfe aus dem strömenden Gemisch.

2. Verfahren (10) nach Anspruch 1, wobei die Länge der Reaktionsleitung (30) und der Massenstrom an Dampf derart sind, um dabei sicherzustellen, dass die Partikel das Ende der Reaktionsleitung (30) in weniger als 1 s erreichen.

3. Verfahren (10) nach Anspruch 1 oder 2, wobei das partikelförmige Material in den Dampfstrom über einen Ejektor (32) an einem Ende der Reaktionsleitung (30) zugeführt wird, wobei der Ejektor (32) eine Eintrittskammer (33) und einen venturiförmigen Austrittskanal (38), eine Düse (36), ein geneigtes Ablenkblech (40) über der Düse (36) und eine Eintrittsöffnung (34) aufweist, durch die das partikelförmige Material auf das Ablenkblech (40) und in die Eintrittskammer (33) zugeführt werden kann, wobei die Düse (36) und der venturiförmige Austrittskanal (38) jeweils mit der Längsachse (31) der Reaktionsleitung (30) ausgerichtet sind, und wobei der Dampf bei einer Temperatur von über 450°C zugeführt wird, um durch die Düse (36) hindurch zu strömen.

4. Verfahren (10) nach Anspruch 3, wobei der Dampf bei einer Temperatur von über 500°C zugeführt wird und der Düse (36) unter einem Druck von kleiner als 1,5 bar(a) zugeführt wird.

5. Verfahren (10) nach einem der vorherigen gehenden Ansprüche, wobei das Abscheiden der Partikel von dem strömenden Gemisch unter Verwendung eines Fliehkraftabscheiders (21) stromabwärts der Reaktionsleitung (30) ausgeführt wird, um die mitgenommenen Partikel zu entfernen und zu sammeln.

6. Verfahren (10) nach einem der vorhergehenden Ansprüche, wobei das Kondensieren der Zucker, des Dampfs und anderer Dünste mittels direkten Kontakts mit einer Kühlflüssigkeit erreicht wird.

7. Verfahren (10) nach einem der vorhergehenden Ansprüche, wobei das Kondensieren der Zucker, des Dampfs und anderer Dünste mittels einer Durchleitung durch zwei nachfolgende Kühlvorrichtungen erreicht wird, wobei die erste als ein Heißdampfkühler wirkt, um die Dampftemperatur auf knapp über den Sättigungspunkt zu senken.

8. Verfahren (10) nach Anspruch 7, wobei die zweite Kühlvorrichtung angeordnet ist, um eine vollständige oder zumindest teilweise Kondensation von allen vorhandenen aus dem Dampf und den Dünsten zu erreichen.

9. Verfahren (10) nach einem der vorhergehenden Ansprüche, wobei das Kondensieren der Zucker, des Dampfs und anderer Dünste mittels Kühlen auf nicht weniger als 60°C erreicht wird, um sicherzustellen, dass eventuell vorhandener Teer ausreichend flüssig bleibt, um keine Verstopfungen zu bilden.

10. Verfahren (10) nach einem der vorhergehenden Ansprüche, wobei ein Lüfter (23) stromabwärts des Kondensators (22) verwendet wird, um einen Gasfluss zu erreichen und um einen Solldruck in der Reaktionsleitung (30) sicherzustellen.

11. Verfahren (10) nach einem der vorhergehenden Ansprüche, wobei der Hemicellulose-Hydrolyse-Schritt Dampf/Wasser bei einer Temperatur zwischen 150°C und 180°C und einem Druck zwischen 6 bar und 10 bar, zum Beispiel bei 165°C und einem Druck von 6,5 bar (Manometer), nach Zugabe einer Säure verwendet.

12. Verfahren (10) nach einem der vorhergehenden Ansprüche, wobei das Abscheiden des partikelförmigen Materials von dem Wasser und der wasserlöslichen Bestandteile nach der Hemicellulose-Hydrolyse einen Filter oder eine Presse verwendet und einen Spül- oder Waschschritt (16) aufweisen kann.

## Revendications

1. Un procédé (10) pour obtenir du sucre à partir de céréales fermentées, comprenant les étapes :
(a) traiter le grain fermenté avec de l'eau chaude pour hydrolyser l'hémicellulose ;
(b) séparer une phase aqueuse contenant les produits de l'hydrolyse de l'hémicellulose du matériau particulaire ;
(c) introduire la matière particulaire dans un flux de vapeur à une température supérieure à 450 °C le long d'un conduit de réacteur (30)
pour former un mélange fluide de particules et de vapeurs, le débit massique de vapeur étant au moins 3 fois le débit massique de matière particulaire, de telle sorte que la matière particulaire est
chauffée rapidement ;
(d) puis séparer au moins 75 % (en masse) des particules du mélange en écoulement après un maximum de 3 secondes ; et
(e) condenser les vapeurs du mélange qui s'écoule.

2. Un procédé (10) selon la revendication 1, dans lequel la longueur du conduit du réacteur (30) et le débit de vapeur sont tels qu'ils garantissent que les particules atteignent l'extrémité du conduit du réacteur (30) en moins de 1 s.

3. Un procédé (10) selon la revendication 1 ou la revendication 2, dans lequel le matériau particulaire est
introduit dans le flux de vapeur à travers un éjecteur (32) à une extrémité du conduit du réacteur (30), l'éjecteur (32) comprenant une chambre d'entrée (33) et un canal de sortie en forme de venturi (38), une buse ( 36), une plaque déflectrice inclinée (40) au-dessus de la buse (36), et un orifice d'entrée (34) à travers lequel le matériau particulaire peut être introduit sur la plaque déflectrice (40) et dans la chambre d'entrée (33), la buse (36) et le canal de sortie en forme de venturi (38) étant tous deux
alignés avec l'axe longitudinal (31) du conduit du réacteur (30) ; et dans lequel la vapeur à une température supérieure à 450 °C est fournie pour s'écouler à travers la buse (36).

4. Un procédé (10) selon la revendication 3, dans lequel la vapeur est fournie à une température supérieure à 500 °C et est introduite dans la buse (36) à une pression inférieure à 1,5 bar (a).

5. Un procédé (10) selon l'une quelconque des revendications précédentes, dans lequel la séparation des particules du mélange en écoulement est effectuée en utilisant un cyclone (21) en aval du conduit du réacteur (30) pour éliminer et collecter les particules entraînées.

6. Un procédé (10) selon l'une quelconque des revendications précédentes, dans lequel la condensation des sucres, de la vapeur et d'autres vapeurs est obtenue par contact direct avec un fluide de refroidissement.

7. Un procédé (10) selon l'une quelconque des revendications précédentes, dans lequel la condensation des sucres, de la vapeur et d'autres vapeurs est obtenue par passage à travers deux dispositifs de refroidissement successifs, le premier agissant comme un désurchauffeur pour abaisser la température de la vapeur juste au-dessus de la point de saturation.

8. Un procédé (10) selon la revendication 7, dans lequel le second dispositif de refroidissement est agencé pour obtenir une condensation totale ou au moins partielle de toute la vapeur et des vapeurs présentes.

9. Un procédé (10) selon l'une quelconque des revendications précédentes, dans lequel la condensation des sucres, de la vapeur et d'autres vapeurs est obtenue par refroidissement à une température non inférieure à 60 °C pour garantir que tout goudron pouvant être présent reste suffisamment fluide pour il ne forme pas de blocages.

10. Un procédé (10) selon l'une quelconque des revendications précédentes, dans lequel un ventilateur (23) en aval du condenseur (22) est utilisé pour obtenir un écoulement de gaz et pour assurer une pression souhaitée dans le conduit du réacteur (30).

11. Un procédé (10) selon l'une quelconque des revendications précédentes, dans lequel l'étape d'hydrolyse de l'hémicellulose utilise de la vapeur/eau à une température comprise entre 150 °C et 180 °C et une pression comprise entre 6 bars et 10 bars, par exemple à 165 °C et une pression de 6,5 bar (jauge), après ajout d'un acide.

12. Un procédé (10) selon l'une quelconque des revendications précédentes, dans lequel la séparation
du matériau particulaire à partir de l'eau et des composés solubles dans l'eau, après hydrolyse de l'hémicellulose, utilise un filtre ou une presse, et peut impliquer une étape de rinçage ou de lavage (16).
